# EUROPEAN PATENT APPLICATION

(11) **EP 4 556 058 A1**
(43) Date of publication of application: **21.05.2025**
(21) Application number: 24212786.8
(22) Date of filing: 13.11.2024
(51) Int. Cl.: A61N 1/375, A61B 17/34, A61N 1/05

(54) **BIOSTIMULATOR HAVING LATERALLY EXTENDING PACING ELECTRODE**

(30) Priority: 14.11.2023 US 202363598920 P; 12.11.2024 US 202418945426
(71) Applicant: Pacesetter, Inc., Sylmar, CA 91342 (US)
(72) Inventor: CHANTASIRIVISAL, Steve, Sylmar, CA 91342 (US); VICTORINE, Keith, Sylmar, CA 91342 (US)
(74) Representative: Barker Brettell Sweden AB

(57) **Abstract**

A biostimulator (100) includes a body (114) having a longitudinal axis (120) and an electronics compartment (302) containing pacing circuitry. The biostimulator (100) includes a header assembly (300) mounted on the body (114) along the longitudinal axis (120). The header assembly (300) includes a header housing (304) having a lateral surface (306) facing laterally outward from the longitudinal axis (120). The header assembly (300) includes an electrode channel (308) in the lateral surface (306). A pacing electrode (112) extends through the electrode channel (308) along an electrode axis (122). The electrode axis (122) is substantially orthogonal to the longitudinal axis (120). Other embodiments are also described and claimed.

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch area (LBBAP) is an alternative to His-bundle pacing. LBBAP involves pacing past the His-bundle toward the right ventricular apex. More particularly, a pacing site for LBBAP is typically below the His-bundle. To achieve optimal results, the pacing site for LBBAP can be mid to high on the interventricular septal wall, in the region close to the tricuspid valve and pulmonary artery outflow track. Furthermore, the pacing site may be at a depth of up to 1.5 cm within the septal wall.

### SUMMARY

Existing leadless pacemakers may not fit, or may interfere with heart structures, when placed at the optimal pacing site for left bundle branch area pacing (LBBAP). More particularly, existing leadless pacemakers have bodies that are long and rigid and, when implanted at the interventricular septal wall, could extend into contact with the cardiac tissue of a ventricular free wall or the tricuspid valve during contraction of the heart. Furthermore, a proximal end of the existing leadless pacemakers may flail within the heart chamber as the heart beats, causing cyclical contact with adjacent heart structures. Such contact could interfere with heart function. Thus, there is a need for a leadless biostimulator that can be engaged to the interventricular septal wall for LBBAP without interfering with adjacent structures of the heart.

A biostimulator is described. In an embodiment, the biostimulator includes a header assembly mounted on a body along a longitudinal axis of the body. The body has an electronics compartment containing pacing circuitry. The header assembly includes a header housing and a pacing electrode. The header housing has a lateral surface facing laterally outward from the longitudinal axis, and an electrode channel in the lateral surface. The pacing electrode extends through the electrode channel along an electrode axis substantially orthogonal to the longitudinal axis.

A biostimulator system is described. In an embodiment, the biostimulator system includes a biostimulator transport system. The biostimulator can be mounted on the biostimulator transport system to carry the biostimulator to or from the target anatomy. A method of pacing using the biostimulator and/or the biostimulator system is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations may have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 illustrates a diagrammatic cross section of a patient heart illustrating an example implantation of a biostimulator in a target anatomy, in accordance with an embodiment.
FIG. 2 illustrates a perspective view of a biostimulator system, in accordance with an embodiment.
FIG. 3 illustrates a perspective view of a biostimulator having a laterally extending electrode in an undeployed state, in accordance with an embodiment.
FIG. 4 illustrates a perspective view of a biostimulator having a laterally extending electrode in a deployed state, in accordance with an embodiment.
FIG. 5 illustrates a top view of a biostimulator having a fixation element in a first position, in accordance with an embodiment.
FIG. 6 illustrates a top view of a biostimulator having a fixation element in a second position, in accordance with an embodiment.
FIG. 7 illustrates a perspective cutaway view of a biostimulator having a header assembly including a drive head, in accordance with an embodiment.
FIG. 8 illustrates a cross-sectional view of a biostimulator having a header assembly including a drive head, in accordance with an embodiment.
FIG. 9A illustrates a top view of a biostimulator including a laterally extended pacing electrode and electrode carrier, in accordance with an embodiment.
FIG. 9B illustrates a top view of a biostimulator including a laterally extended pacing electrode and a partially retracted electrode carrier, in accordance with an embodiment.
FIG. 9C illustrates a top view of a biostimulator including a laterally extended pacing electrode and a fully retracted electrode carrier, in accordance with an embodiment.
FIG. 10A illustrates a pictorial view of a biostimulator having radiopaque markers misaligned in a projection view, in accordance with an embodiment.
FIG. 10B illustrates a pictorial view of a biostimulator having radiopaque markers aligned in a projection view, in accordance with an embodiment.
FIG. 11 illustrates a flowchart of a method of using a biostimulator to pace target tissue, in accordance with an embodiment.

### DETAILED DESCRIPTION

Embodiments describe a biostimulator and a biostimulator system for pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a component of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator having a laterally extending pacing electrode is provided. More particularly, a body of the biostimulator can extend along a longitudinal axis, and the pacing electrode can extend along an electrode axis substantially orthogonal to the longitudinal axis. The substantial orthogonality of the biostimulator body and pacing electrode allows the body to lie parallel to a septal wall when the pacing electrode extends into the septal wall. Accordingly, the body can be directed toward or fit within a ventricular apical space, avoiding interference with the tricuspid valve or contact with the ventricular free wall. While the body is so placed for long term implant stability, the pacing electrode can extend into the mid or high septal wall to perform left bundle branch area pacing (LBBAP).

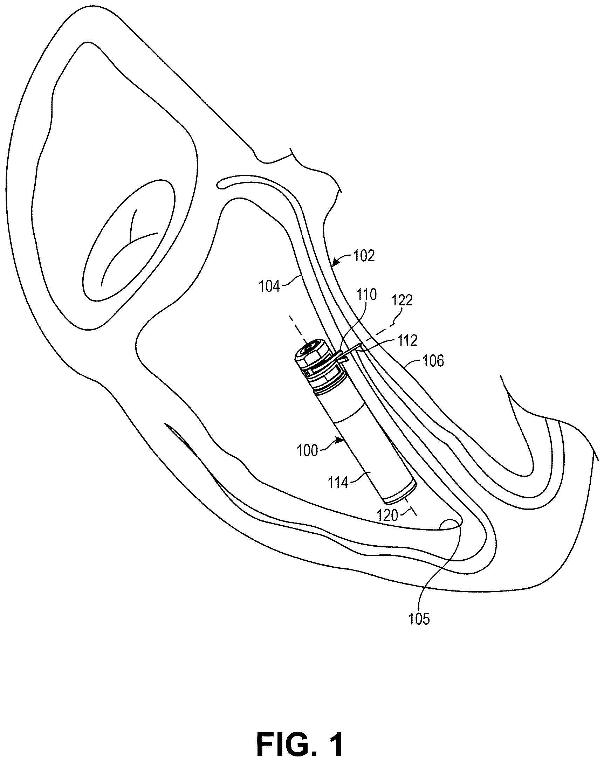

Referring to FIG. 1, a diagrammatic cross section of a patient heart illustrating an example implantation of a biostimulator in a target anatomy is shown in accordance with an embodiment. The diagram shows a biostimulator 100 attached to a patient heart 102 with a body 114 of the biostimulator 100 positioned toward a ventricular apex 105. A leadless biostimulator system, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or a right ventricle of the heart 102, or attached to an inside or outside of the cardiac chamber. For example, the biostimulator 100 can be attached to one or more of an interventricular septal wall 104 or a ventricular apex 105 of the heart 102. More particularly, the biostimulator 100 can be delivered to an interventricular septum, and one or more elements, such as a fixation element 110 or a pacing electrode 112, can pierce the interventricular septal wall 104 of the septum to engage and anchor the biostimulator 100 to a target tissue 106. A body 114 of the biostimulator 100 can be directed toward the ventricular apex 105, or otherwise positioned orthogonal to the pacing electrode 112, as described below.

The body 114 of the biostimulator 100 can have a longitudinal axis 120 and the pacing electrode 112 can extend along an electrode axis 122. The electrode axis 122 can be substantially orthogonal to the longitudinal axis 120. As used herein, the terms "orthogonal" or "substantially orthogonal" are intended to encompass a range of angles including 90 degrees. The range of angles is not limited to 90 degrees, however, and can include a workable range of angles including 75 to 115 degrees, e.g., 80 to 100 degrees or 85 to 95 degrees. It will be appreciated that such orthogonality (or substantial orthogonality) can be measured within a conical volume having a central angle axis set at 90 degrees to the longitudinal axis 120, and a conical region swept out by sides set at the range of angles from the central angle axis. Accordingly, whereas the electrode axis 122 can extend orthogonal (and into) to the septal wall 104 when the biostimulator 100 is affixed to the septal wall 104 and inserted into the target tissue 106, the longitudinal axis 120 can be parallel to (and along) a wall surface of the septal wall 104. For example, the longitudinal axis 120 can be directed toward an apex wall of the ventricular apex 105 and a proximal end of the body 114 may be located at the apex.

When the biostimulator 100 is delivered to and plunged into the septum of the heart 102, the pacing electrode 112 may be positioned for deep septal pacing at a target anatomy, such as at a bundle branch in the septum. For example, for LBBAP, the pacing electrode 112 can be positioned at a left bundle branch in the septum. The biostimulator 100 may deliver pacing impulses through the pacing electrode 112 to the target anatomy. Accordingly, the pacing electrode 112 can be located to effectively probe and pace the target anatomy, while the body 114 can be placed in a safe and non-obstructive location within the heart chamber.

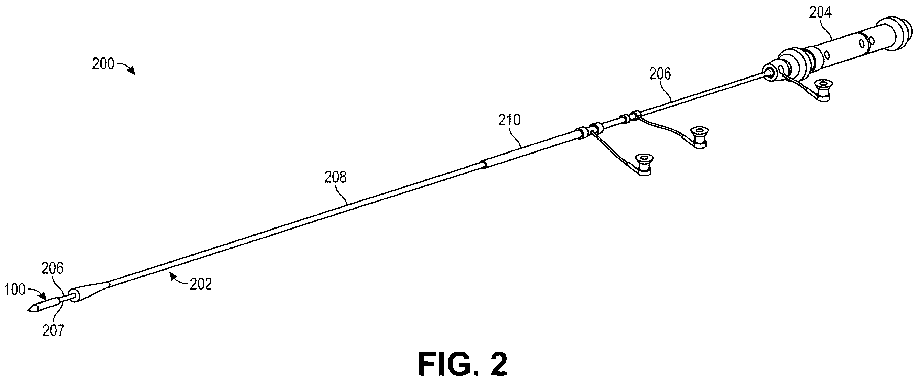

Referring to FIG. 2, a perspective view of a biostimulator system is shown in accordance with an embodiment. Leadless pacemakers or other leadless biostimulators can be delivered to or retrieved from a patient using delivery or retrieval systems. The leadless biostimulator system can include delivery or retrieval systems, which may be catheter-based systems used to carry a leadless biostimulator intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator 100 can be mounted on a distal end of a catheter of the biostimulator transport system 202. The biostimulator 100 can thereby be advanced intravenously into or out of the heart 102.

The biostimulator transport system 202 can include a handle 204 to control movement and operations of the transport system from outside of a patient anatomy. One or more elongated members extend distally from the handle 204. For example, a support member 206 can extend distally from the handle 204. The support member 206 can extend to a distal end of the transport system. In an embodiment, the biostimulator 100 is mounted on the biostimulator transport system 202, e.g., at a distal end of the support member 206.

In an embodiment, the biostimulator transport system 202 includes a drive shaft 207 that can engage the biostimulator 100. For example, the drive shaft 207 can engage a torque feature of the biostimulator 100 to laterally extend or retract the pacing electrode 112 relative to the body 114, as described below.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternate components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy, and actuation of the fixation element 110 and/or pacing electrode 112, as described below, during implantation of the biostimulator 100 at the target anatomy. Accordingly, the biostimulator transport system 202 can incorporate features to retain and actuate the biostimulator 100.

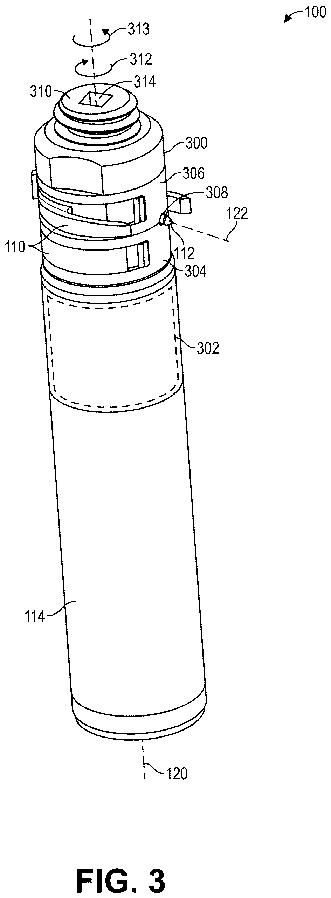

Referring to FIG. 3, a perspective view of a biostimulator having a laterally extending electrode in an undeployed state is shown in accordance with an embodiment. The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within the body 114 of the biostimulator 100 for pacing the cardiac chamber upon receiving a triggering signal from at least one other device within the patient anatomy. The two or more electrodes of the biostimulator 100 can include the pacing electrode 112 that is extendable in a radially outward direction, relative to the body 114, to act as an active electrode within the target tissue 106. The electrodes can deliver pacing pulses to target anatomies, such as bundle branches within the septum of the heart 102, to perform pacing, e.g., deep septal pacing, and optionally, can sense electrical activity from the muscle. The electrodes may also communicate bidirectionally with at least one other device within or outside the patient anatomy.

The body 114 has the longitudinal axis 120, e.g., a body axis. The body 114 can contain a primary battery to provide power for pacing, sensing, and communication, which may include, for example, bidirectional communication. The body 114 can optionally have an electronics compartment 302 (shown by hidden lines) to hold circuitry adapted for different functionality. For example, the electronics compartment 302 can contain pacing circuitry for sensing cardiac activity from the electrodes, for receiving information from at least one other device via the electrodes, for generating pacing pulses for delivery to tissue via the pacing electrode 112, or other circuitry. Accordingly, the pacing circuitry can be electrically connected to the pacing electrode 112. The electronics compartment 302 may contain circuits for transmitting information to at least one other device via the electrodes and can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

In an embodiment, the biostimulator 100 includes a header assembly 300 mounted on the body 114 along the longitudinal axis 120. The header assembly 300 can include components, e.g., one or more fixation elements 110, to affix the biostimulator 100, including the body 114, to the target tissue 106. The header assembly 300 may also include components, e.g., the pacing electrode 112, to deliver pacing impulse to the target tissue 106.

The header assembly 300 can include a header housing 304 mounted on the body 114. For example, the header housing 304 can be directly located on the portion of the body 114 that contains the electronics compartment 302. The header housing 304, like the body 114, may have a partly cylindrical outer surface surrounding the longitudinal axis 120. Accordingly, the header housing 304 can have a lateral surface 306 facing laterally outward from the longitudinal axis 120.

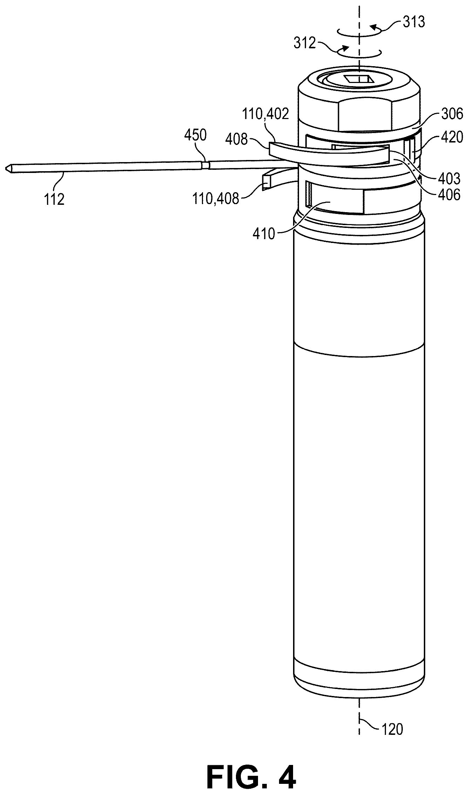

In an embodiment, the header housing 304 has an electrode channel 308 in the lateral surface 306. The electrode channel 308 can include a hole in the lateral surface 306 that connects an internal volume of the header housing 304 with a surrounding environment. The pacing electrode 112 can be extendable and/or retractable within the electrode channel 308. More particularly, the pacing electrode 112 can extend from an undeployed state (FIG. 3) to a deployed state (FIG. 4). Accordingly, in the deployed state, the pacing electrode 112 can extend through the electrode channel 308, along the electrode axis 122, into the target tissue 106 within the surrounding environment.

The header assembly 300 can include a drive head 310 mounted within the header housing 304. The drive head 310 structure and function is described in more detail below. It will be appreciated, however, that the drive head 310 can be rotated in a first circumferential direction 312 about the longitudinal axis 120 relative to the header housing 304 to extend the pacing electrode 112 through the electrode channel 308. By contrast, the drive head 310 can be rotated in a second circumferential direction 313 about the longitudinal axis 120, opposite to the first direction, to retract the pacing electrode 112 through the electrode channel 308.

Rotation of the drive head 310 may be caused by the drive shaft 207 of the biostimulator transport system 202. More particularly, the drive shaft 207 can engage a torque feature 314 of the drive head 310 to transmit torque from the biostimulator transport system 202 to the drive head 310. For example, a user can rotate a knob of the handle 204 to input torque to the drive shaft 207, and the drive shaft 207 may have a distal portion sized and shaped to engage the torque feature 314 of the drive head 310. The torque feature 314 may be a recess having a non-circular cross-sectional profile, e.g., a square or hex-shaped socket. The distal portion of the drive shaft 207 can include a square or hex-shaped key that fits in the socket. Accordingly, torque feature 314 can receive and mate with the distal portion of the drive shaft 207. The user input torque may therefore be transmitted through the shaft to the torque feature 314 to actuate the drive head 310, e.g., via rotation in the first circumferential direction 312 or the second circumferential direction 313.

The biostimulator 100 can include one or more of the fixation elements 110 mounted on the lateral surface 306. More particularly, several fixation elements 110 can be mounted on the lateral surface 306. The fixation elements 110 can be actuated to cause the elements to move relative to the header housing 304 and engage the target tissue 106, as described below.

Referring to FIG. 4, a perspective view of a biostimulator having a laterally extending electrode in a deployed state is shown in accordance with an embodiment. Each fixation element 110 of the biostimulator 100 can include a claw 402 extending laterally outward from the lateral surface 306. The claw 402 can have a slender, curved structure. The claw 402 can curve outward from a claw origin 403 at a claw base 406 to a claw tip 408. For example, the claw 402 can extend in the first circumferential direction 312 about the longitudinal axis 120 from the claw origin 403 to the claw tip 408. Similarly, another claw 402 can curve or extend in the second circumferential direction 313 about the longitudinal axis 120 from a respective claw origin 403 to a respect claw tip 408. Each claw 402 may therefore have respective sharp claw tips 408 that can penetrate the target tissue 106 when the claw 402 is pressed against the septal wall 104.

Each claw 402 can be slidable on the lateral surface 306. More particularly, each claw 402 can be slidable on the lateral surface 306 about the longitudinal axis 120 to move the claw tip 408. When the lateral surface 306 is against the wall surface of the septal wall 104, the claws 402 can penetrate and hook into the target tissue 106 to affix the biostimulator 100 to the septal wall 104.

In an embodiment, the opposing mechanical anchors provided by the claws 402 can be rotated about the longitudinal axis 120 to affix the biostimulator 100 to the ventricular septum. Alternatively, one anchor can be fixed on the header housing 304, and the body 114 can be rotated to drive the fixed claw into the target tissue 106. The other anchor may then be rotated about the longitudinal axis 120 to secure the target tissue 106 between the inward facing curves of the claws 402.

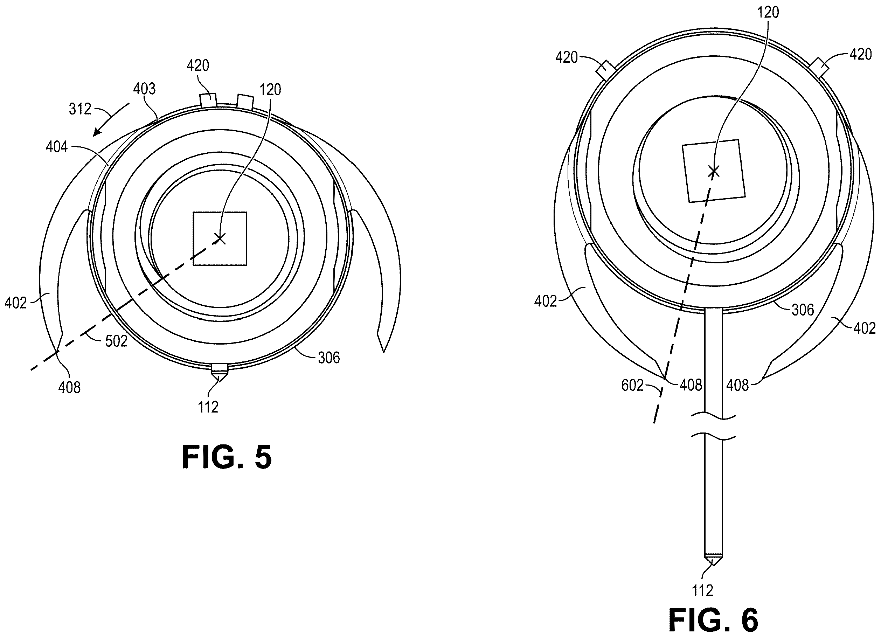

In an embodiment, the claw 402 includes the claw base 406 mounted within a claw channel 410 of the lateral surface 306. More particularly, the claw channel 410 may be a groove or recess in the lateral surface 306. The claw channel 410 can extend along at least a portion of a circumference of the lateral surface 306. For example, the claw base 406 may include a c-shaped strip or band of material that clips onto the circumferential surface in the claw channel 410. The c-shaped strip can therefore be retained on the lateral surface 306 and may be capable of sliding over the circumferential surface, about the longitudinal axis 120. As the claw base 406 slides, the claw 402, which extends laterally outward from the claw base 406, may also move about the longitudinal axis 120. More particularly, the claw 402 is slidable on the lateral surface 306 about the longitudinal axis 120 to move the claw tip of the claw 402 from a first radial location 502 relative to the longitudinal axis 120 (FIG. 5) to a second radial location 602 relative to the longitudinal axis 120 (FIG. 6).

Sliding of the claw bases 406 within the claw channels 410 may be caused by actuation of a claw tab 420 of the claw structure. More particularly, the claw tab 420 can be a tab of material extending outward from the claw base 406 at a location that is circumferentially offset from the claw origin 403. For example, the claw 402 can extend from the claw origin 403 at a first location on the claw base 406 about the longitudinal axis 120 in the first circumferential direction 312, and the claw tab 420 can be located on the claw base 406 at a second location separated from the first location in the second circumferential direction 313 about the longitudinal axis 120. The claw tab 420 can include a nub or protrusion that can be engaged, e.g., by a catheter or a shaft of the biostimulator transport system 202, and pushed to move the claw base 406 around the lateral surface 306. More particularly, the claw tabs 420 can be pushed to rotate the claws 402 into place within the target tissue 106.

Referring to FIG. 5, a top view of a biostimulator having a fixation element in a first position is shown in accordance with an embodiment. In the top view, the circumferential offset between the claw origin 403 and the claw tab 420 is evident. The claw tab 420 can be pushed in the first circumferential direction 312 to move the claw tab 420 around the lateral surface 306 into the target tissue 106. In the undeployed state shown in FIG. 5, the claw tip 408 can be at a first radial location 502 relative to the longitudinal axis 120.

The claw 402 can be formed from a flexible material, such as a shape-memory alloy, which allows the curved prong to be pushed or biased radially inward against the lateral surface 306, but to recover to the laterally outward position when the biasing load is removed. Accordingly, the biostimulator 100 can be loaded into an interior of a catheter, such as the protective sleeve 208, that has a smaller inner diameter than a distance between the claw tips 408. The claws 402 can be squeezed down against the lateral surface 306 by the catheter. Upon removal or retraction of the catheter, however, the claws 402 can spring outward to the position illustrated in FIG. 5.

Referring to FIG. 6, a top view of a biostimulator having a fixation element in a second position is shown in accordance with an embodiment. When the claws 402 spring outward and the claw tab 420 is pushed, the claw 402 can slide about the longitudinal axis 120. The claw 402 can rotate into the tissue. More particularly, the claw tip 408 can advance to a second radial location 602 relative to the longitudinal axis 120. Tissue can become trapped between the lateral surface 306 of the header housing 304 and/or body 114 and the inner surface of the claw 402. Accordingly, the claw 402 can side-stitch the target tissue 106 to anchor the biostimulator 100 against the septal wall 104.

One or more delivery system features can facilitate deployment of the claw 402. In an embodiment, the biostimulator transport system 202 includes posts to engage the claw tabs 420. The posts may be moved about the longitudinal axis 120, e.g., via a handle control feature, to cause the claw bases 406 (see FIG. 4) to slide along the lateral surface 306. As the claw bases 406 slide along the lateral surface 306 in opposite directions, the opposing claw tips 408 can engage and pierce the tissue. The pierced tissue can become trapped between the inner surfaces of the claws 402 and the lateral surface 306 of the biostimulator housing. Accordingly, the biostimulator 100 may be anchored to the target tissue 106.

An alternative delivery system feature may include a tubular component that can be forced through a gap separating the claw tabs 420. The tubular component may, for example, have a side wall that extends and tapers to a point. The point can be located between the gap that separates the claw tabs 420. Axial actuation of the tubular component, e.g., sliding the tubular component distally relative to the claw tabs 420, can drive a tapered segment of the sidewall through the gap. At each axial location of the tapered segment, the sidewall segment can widen and therefore force the claw tabs 420 apart. More particularly, axial movement of the tubular component can increase the gap between the claw tabs 420. The claw tabs 420 are therefore driven apart from each other and the respective claw bases 406 of the claws 402 slide in opposite directions along the lateral surface 306. The claw tips 408 can therefore engage the target tissue 106 to anchor the biostimulator 100 within the target anatomy.

The biostimulator 100 may include features to control movement of the claws 402. As described above, the claw channel 410 in the header housing 304 can guide claw base 406 over a predetermined path, e.g., circumferentially along the lateral surface 306. In addition to controlling the direction of sliding of the claw base 406, the claw channel 410 can include one or more detents that engage corresponding detents of the claw base 406. For example, a recess may be formed within the claw channel 410 that a protrusion on the claw base 406 can snap into. When the claw base 406 snaps into the detent, further movement of the claw base 406 may be resisted. The claw 402 may therefore be held in place. When the detent is located such that the claw base 406 engages the detent when the claw tip 408 is at the second radial location 602, claw 402 can be held in place with the target tissue 106 trapped between the claw 402 and the lateral surface 306. Accordingly, detents within the claw channel 410 can hold the claws 402 in place with the biostimulator 100 anchored against the target tissue 106.

The description of the claw channel 410 above is illustrative and not limiting. For example, rather than having a c-shaped profile, the claw base 406 may be shortened. A length of the claw base 406 may be insufficient to clip on to the circumferential lateral surface 306. The claw channel 410 may therefore retain the claw base 406 using one or more grooves to receive corresponding tabs of the claw base 406. For example, the claw channel 410 can have a y-shaped cross-sectional profile with a ridge extending over the channel. The claw base 406 may fit within the y-shaped channel, and the ridge can hold the claw base 406 in place. Accordingly, the claw channel 410 and/or claw base 406 may be sized and shaped to interface with each other such that the claw 402 is retained against the header housing 304, and the claw 402 is guided along a path to allow the claw tip 408 to pierce the target tissue 106 and anchor the biostimulator 100 against the target tissue 106.

Referring again to FIG. 5, the pacing electrode is shown in the undeployed state. In the undeployed state, the biostimulator can be delivered against the septal wall. A pacing tip of the pacing electrode 112 can sit against the septal wall 104. Referring again to FIG. 6, when the claws 402 pierce and anchor within the target tissue 106, the pacing electrode 112 can be extended. More particularly, the pacing electrode 112 is shown in the deployed state (see also FIG. 1). In the deployed state, the pacing tip of the pacing electrode 112 can plunge into the target tissue 106 toward an intended pacing site, such as a left bundle branch. Extension and retraction of the pacing electrode 112 can be driven by components of the header assembly 300, as described below.

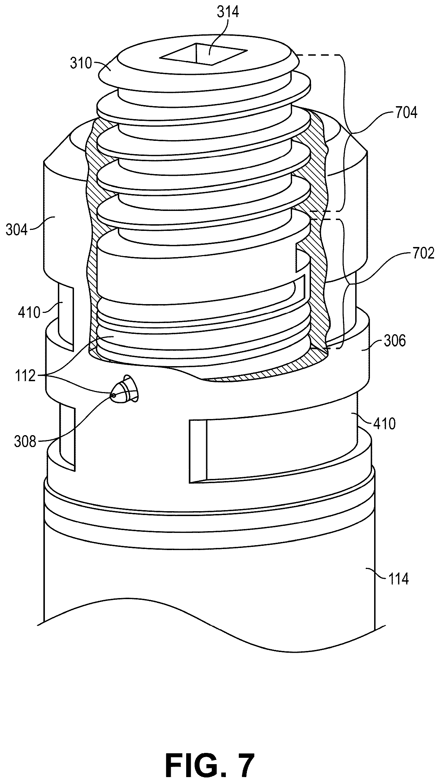

Referring to FIG. 7, a perspective cutaway view of a biostimulator having a header assembly including a drive head is shown in accordance with an embodiment. The drive head 310, which is received within the header housing 304, can include a spool portion 702 and a threaded portion 704. In an embodiment, the pacing electrode 112 is wound about the spool portion 702. For example, the spool portion 702 can include a helical channel extending along an outer surface of the drive head 310. The helical channel can have a width to receive the pacing electrode 112. Accordingly, the pacing electrode 112 can wind along an outer surface of the drive head 310. The pacing electrode 112 may therefore pass through the electrode channel 308 in the lateral surface 306 of the header housing 304 to enter the helical channel on the spool portion 702 of the drive head 310, and to wind along the helical channel. The electrode can therefore be wound on the spool portion 702, which acts as a reel, and then exit through the hole in the header housing 304 to extend into the surrounding environment in the undeployed state or the deployed state.

The threaded portion 704 of the drive head 310 may be threadably coupled to the header housing 304. For example, the threaded portion 704 can include an external thread extending helically about the drive head 310, and the threaded portion 704 can engage a corresponding internal thread of the drive housing. Accordingly, when the drive head 310 is rotated, e.g., by rotating the torque feature 314 with the drive shaft 207 of the biostimulator transport system 202, the threaded portion 704 can advance or retract within an internal channel of the drive head 310. More particularly, rotation of the drive head 310 in a first direction can cause the drive head 310 to advance within the internal channel, and rotation of the drive head 310 in an opposite direction can retract the drive head 310 within the internal channel.

When the drive head 310 is twisted to rotate the threaded portion 704, the spool portion 702 can be correspondingly rotated. Rotation of the spool portion 702 can advance the pacing electrode 112 through the electrode channel 308. Accordingly, driving the drive head 310 to rotate about the longitudinal axis 120 can cause the pacing electrode 112 to unwind from the spool portion 702 and advance laterally outward through the lateral surface 306 along the electrode axis 122 into the target tissue 106.

In FIG. 7, the claws 402 of the biostimulator 100 are shown detached, or omitted, from the header assembly 300. Accordingly, the claw channels 410 can be shown. The claw channels 410 can be c-shaped grooves in the lateral surface 306 within which the claw bases 406 can be clipped to retain and guide the claws 402.

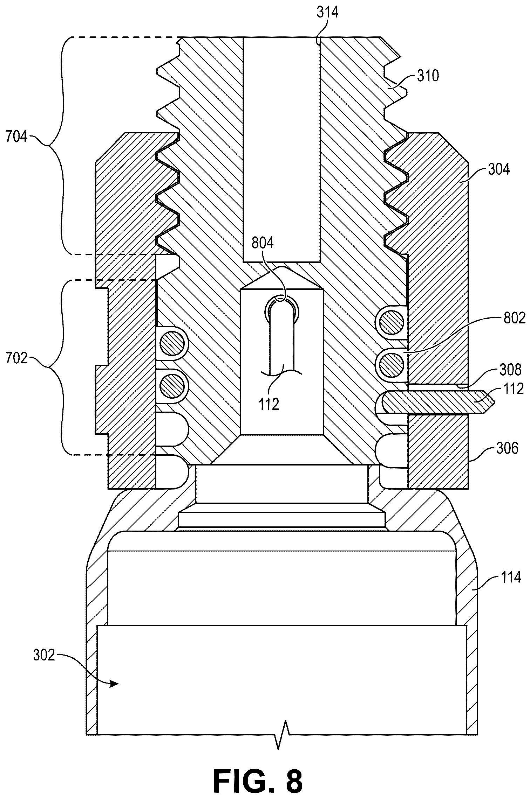

Referring to FIG. 8, a cross-sectional view of a biostimulator having a header assembly including a drive head is shown in accordance with an embodiment. A cross-sectional view of the header assembly 300 reveals the pacing electrode 112 path within the drive head 310. The pacing electrode 112 can extend from the pacing tip, through the electrode channel 308, into an internal volume of the header housing 304. More particularly, a spool channel 802 formed between an outer surface of the spool portion 702 of the drive head 310 and an inner surface of the header housing 304 can receive the pacing electrode 112 along a spiral or helical path. Accordingly, a top region of the biostimulator 100 can contain the extendable electrode with a pacing tip that is wound and protected in the biostimulator housing during delivery.

The drive head 310 can include an electrode port 804. The pacing electrode 112 can spiral through the spool channel 802 to a proximal location at which the pacing electrode 112 extends and passes through the electrode port 804. The electrode port 804 can be a hole formed in the drive head 310. The hole can connect the spool channel 802 to an internal volume of the drive head 310. The internal volume may be located along the longitudinal axis 120 above the electronics compartment 302. Accordingly, the pacing electrode 112, or an electrical lead connected to the pacing electrode 112, can extend into the electronics compartment 302 to electrically connect the pacing electrode 112 to electronic circuitry within the electronics compartment 302.

The pacing electrode 112 can be extended by rotating the drive mechanism at the top of the biostimulator 100, e.g., via torquing of the torque feature 314, until a target penetration depth is reached. In an embodiment, the pacing electrode 112 can be formed from a flexible elongate element, such as a metallic cable or braid. Alternatively, as described below, the pacing electrode 112 may have a two-part construction, including a core wire and a carrier tube.

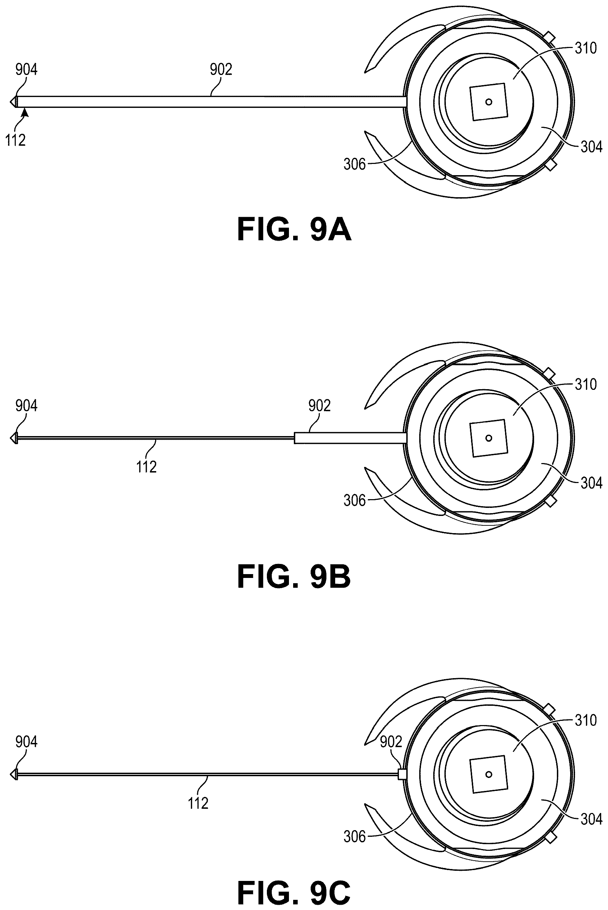

Referring to FIG. 9A, a top view of a biostimulator including a laterally extended pacing electrode and electrode carrier is shown in accordance with an embodiment. The biostimulator 100 may include an electrode carrier 902. Electrode carrier 902 can be tubular, and the pacing electrode 112 may extend through a carrier lumen of the electrode carrier 902. For example, the electrode carrier 902 can be a tube around a wire forming a portion of the pacing electrode 112. The carrier lumen can be a central lumen extending longitudinally through the electrode carrier 902 and, thus, is not visible in the top view. It will be appreciated, however, that the pacing electrode 112 extends from the pacing tip through the pacing lumen and the lateral surface 306 into the header housing 304 and the drive head 310.

The electrode carrier 902 may be formed from an insulative material, such as a polymer. Alternatively, the electrode carrier 902 can include a tubular braiding having an insulative coating, e.g., a parylene or an ethylene tetrafluoroethylene (ETFE) coating, on an inner surface surrounding the pacing electrode 112. Other insulative structures, such as an insulative ring 904 longitudinally between the pacing tip and the electrode carrier 902, may also be incorporated in the electrode design to insulate the pacing electrode 112 from the electrode carrier 902. The insulative ring 904 can be an annular insulator, e.g., a ring of polyether ether ketone (PEEK), that separates the pacing tip from a distal end of the electrode carrier 902. Accordingly, the electrode carrier 902 can be electrically insulated from the pacing electrode 112, and can electrically insulate the pacing electrode 112 from surrounding structures.

Advancement of the pacing electrode 112 and electrode carrier 902 can be achieved by rotating the drive head 310, as described above. Rotation of the drive head 310 can advance the components from the spool portion 702 through the electrode channel 308 into the deployed state shown in FIG. 9A. When the components are fully advanced, the pacing tip can be located at the pacing site.

Referring to FIG. 9B, a top view of a biostimulator including a laterally extended pacing electrode and a partially retracted electrode carrier is shown in accordance with an embodiment. In an alternative or additional embodiment, the electrode carrier 902 can be formed from a shape memory material. For example, the electrode carrier 902 tube may include a nickel titanium alloy that is shape set to assume a straightened configuration when extended from the spool portion 702 through the electrode channel 308. The characteristics of the shape memory material used to form the electrode carrier 902 allows the pacing electrode 112 to be carried from the wound configuration in the header housing 304 to a straight configuration when extended out of the header housing 304. The pacing tip can therefore be carried deep into the septal wall 104 orthogonal to the longitudinal axis 120 of the biostimulator 100 and the wall surface of the septal wall 104. The pacing tip, which is connected to the pacing circuitry through the pacing electrode 112 and one or more leads or electrical feedthroughs can deliver pacing impulses from the pacing circuitry to the target tissue 106.

The pacing electrode 112 may include, in addition to the pacing tip, one or more alternate electrodes. For example, the alternate electrodes can be placed at various distances along the pacing electrode 112 or the electrode carrier 902. Referring again to FIG. 4, one such alternate electrode 450 is shown proximal to the pacing tip along the pacing electrode 112. The alternate electrode 450 can be used to sense or pace tissue proximal to the pacing site being stimulated by the pacing tip.

Referring again to FIG. 9B, the electrode carrier is shown in a partially retracted configuration relative to the pacing tip. The electrode carrier 902, like the pacing electrode 112, can extend through the spool channel 802 to the electrode port 804 (see FIG. 8). The electrode carrier 902 may have a proximal bend that extends through the electrode port 804 and hooks into the internal volume of the drive head 310. The proximal portion of the electrode carrier 902 may therefore be secured relative to the drive head 310 at the electrode channel 308. By contrast, the pacing electrode 112 may slide freely through the electrode carrier 902 and may not be fixed to the drive head 310 at the electrode port 804.

The pacing tip can have a spear tip including a distal point and proximal cleats. The cleats can resist backward movement through the target tissue 106. More particularly, the electrode tip can, when engaged to the target tissue 106, resist being pulled out of the tissue. Accordingly, when the drive head 310 is rotated to wind up and retract the electrode carrier 902, the pacing tip can remain in place at the target pacing site.

Referring to FIG. 9C, a top view of a biostimulator including a laterally extended pacing electrode and a fully retracted electrode carrier is shown in accordance with an embodiment. When the electrode carrier 902 is fully retracted, the wire of the pacing electrode 112 can extend laterally from the lateral surface 306 to the pacing tip. The pacing electrode 112, which may be formed from a thin wire, coil, or braid, may have excellent fatigue resistance. Leaving the pacing electrode 112 in place can therefore allow the tip to reliably pace the target tissue 106 while providing excellent implant stability. The biostimulator 100 may be retained and anchored in place at the septal wall 104 by the deployed claws 402.

Although the pacing electrode 112 has been described as being internal to the electrode carrier 902 above, the physical locations of those components may be reversed. For example, optionally, the electrode carrier 902 may be an internal stylet that extends through an inner lumen of an electrode coil or braid. The coil or braid can form the pacing electrode 112 and can be pushed laterally outward into the target tissue 106 by the stylet. Accordingly, the electrode design described above is illustrative and not restrictive.

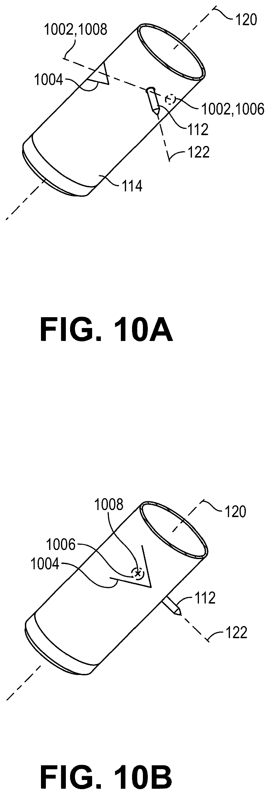

Referring to FIG. 10A, a pictorial view of a biostimulator having radiopaque markers misaligned in a projection view is shown in accordance with an embodiment. To ensure that the pacing electrode 112, when deployed, extends laterally in a direction of the septal wall 104, and not away from the septal wall 104, the biostimulator 100 can include a marker system to aid in orienting the biostimulator 100 under medical imaging. In an embodiment, the biostimulator 100 includes several radiopaque markers 1002 mounted on the body 114. For example, the radiopaque markers 1002 can include a first marker 1004 on a first side of body 114, and a second marker 1006 on a second side of the body 114 opposite to the first side. The radiopaque markers 1002 can be aligned along the transverse axis 1008 of the body 114. The transverse axis 1008 may be orthogonal to the electrode axis 122 along which the pacing electrode 112 extends laterally from the header housing 304. Accordingly, the markers may be aligned with each other in a direction that is orthogonal to the electrode axis 122.

Referring to FIG. 10B, a pictorial view of a biostimulator having radiopaque markers aligned in a projection view is shown in accordance with an embodiment. The radiopaque markers 1002, one on the front and one on the back of the body 114, can be aligned under fluoroscopy to orient the biostimulator 100 so that the pacing electrode 112 and the fixation elements 110 (see FIGS. 1, 3, 4) are facing the septal wall 104. Accordingly, when the markers are aligned in a first projection, and the projection view is along or parallel to the septal wall 104, the pacing electrode 112 can be assumed to be directed orthogonal to the septal wall 104. The pacing electrode 112 can therefore be deployed into the septal wall 104 via rotation of the drive head 310.

In an embodiment, the radiopaque markers 1002 on opposite sides of the body 114 can act as sites to properly orient the biostimulator 100. For example, the first marker 1004 may have a v-notch profile. The v-notch profile includes two lines slanting to a point. The second marker 1006 may have a bead profile. The bead profile may include a circular shape, e.g., a dot. The bead profile can fit within the v-notch profile to align the markers in the visual projection. More particularly, when the projection shows the dot within a crux of the v-notch, the transverse axis 1008 can be determined to be extending toward the imager, and the electrode axis 122 can be determined to be orthogonal to the transverse axis 1008 and directed toward a septal wall 104 that is seen parallel to the transverse axis 1008, e.g., as shown in FIG. 1. An appropriate device orientation can therefore be confirmed and the claws 402 can be deployed to anchor the biostimulator 100 against the septal wall 104. Similarly, the pacing electrode 112 can be advanced to engage the target tissue 106 for pacing.

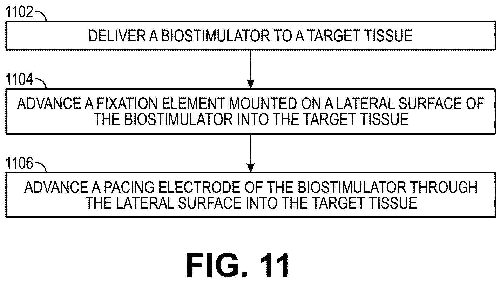

Referring to FIG. 11, a flowchart of a method of using a biostimulator to pace target tissue is shown in accordance with an embodiment. At operation 1102, the biostimulator 100 is delivered to the target tissue 106. The biostimulator transport system 202 can be advanced through the anatomy until the biostimulator 100 is near the septal wall 104. More particularly, a base of the biostimulator 100 may be oriented toward the apex of the heart chamber, and the body 114 of the biostimulator 100 can be parallel with the septal wall 104. The biostimulator 100 may then be viewed under medical imaging, e.g., fluoroscopy, and the marker system can be aligned to orient the biostimulator 100 such that electrode axis 122 is orthogonal to the septal wall 104.

In operation 1104, the fixation element 110 mounted on the lateral surface 306 of the biostimulator 100 can be advanced into the target tissue 106. For example, as described above, the claw tabs 420 can be actuated to slide the claws 402 forward into the septal wall 104. The claws 402 can pierce and engage the target tissue 106, anchoring the biostimulator 100 against the wall surface of the septal wall 104. The claws 402 may be locked in place, e.g., by detents within the claw channels 410. Accordingly, the biostimulator 100 may be secured in place with the electrode axis 122 directed forward, orthogonal to the septal wall 104, and in a direction of the pacing site.

At operation 1106, the pacing electrode 112 is advanced through the lateral surface 306 of the biostimulator 100 into the target tissue 106. Advancing the pacing electrode 112 can include rotating the drive head 310 to advance the pacing electrode 112 held in the spool portion 702 of the drive head 310. For example, the drive shaft 207 of the biostimulator transport system 202 can torque the drive head 310 to rotate the spool portion 702 and unwind the pacing electrode 112 or the electrode carrier 902 through the electrode channel 308. The pacing tip can advance through the target tissue 106 to the desired depth at the pacing site. The biostimulator 100 may then capture LBBAP.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a body (114) having a longitudinal axis (120) and an electronics compartment (302) containing pacing circuitry; and
a header assembly (300) mounted on the body (114) along the longitudinal axis (120), wherein the header assembly (300) includes
a header housing (304) having a lateral surface (306) facing laterally outward from the longitudinal axis (120), and an electrode channel (308) in the lateral surface (306), and
a pacing electrode (112) extending through the electrode channel (308) along an electrode axis (122) substantially orthogonal to the longitudinal axis (120).

2. The biostimulator of claim 1, wherein the header assembly (300) includes a drive head (310) mounted within the header housing (304), wherein the drive head (310) includes a spool portion (702) and a threaded portion (704), wherein the pacing electrode (112) is wound about the spool portion (702), and wherein the threaded portion (704) is threadably coupled to the header housing (304).

3. The biostimulator of claim 2, wherein the drive head (310) includes an electrode port (804), and wherein the pacing electrode (112) extends through the electrode port (804).

4. The biostimulator of claim 2 or 3, wherein rotation of the drive head (310) in a first direction relative to the header housing (304) extends the pacing electrode (112) through the electrode channel (308), and wherein rotation of the drive head (310) in a second direction relative to the header housing (304) retracts the pacing electrode (112) through the electrode channel (308).

5. The biostimulator of any one of claims 1 to 4, further comprising a fixation element (110) mounted on the lateral surface (306).

6. The biostimulator of claim 5, wherein the fixation element (110) includes a claw (402) extending laterally outward from the lateral surface (306), and wherein the claw (402) is slidable on the lateral surface (306).

7. The biostimulator of claim 6, wherein the claw (402) is slidable on the lateral surface (306) about the longitudinal axis (120) to move a claw tip (408) of the claw (402) from a first radial location (502) relative to the longitudinal axis (120) to a second radial location (602) relative to the longitudinal axis (120).

8. The biostimulator of claim 6 or 7, wherein the claw (402) includes a claw base (406) mounted within a claw channel (410) of the lateral surface (306), and wherein the claw (402) extends laterally outward from the claw base (406).

9. The biostimulator of claim 8, wherein the claw (402) extends in a first circumferential direction (312) about the longitudinal axis (120) from a claw origin (403) at the claw base (406), and wherein the claw (402) includes a claw tab (420) at a location on the claw base (406) in a second circumferential direction (313) about the longitudinal axis (120).

10. The biostimulator of any one of claims 1 to 9, further comprising a plurality of radiopaque markers (1002) mounted on the body (114), wherein the plurality of radiopaque markers (1002) are aligned along a transverse axis (1008), and wherein the transverse axis (1008) is orthogonal to the electrode axis (122).

11. The biostimulator of claim 10, wherein the radiopaque markers (1002) include a first marker (1004) having a v-notch profile.

12. The biostimulator of claim 11, wherein the radiopaque markers (1002) include a second marker (1006) having a bead profile.

13. The biostimulator of any one of claims 1 to 12, wherein the header assembly (300) includes an electrode carrier (902), and wherein the electrode carrier (902) is tubular and the pacing electrode (112) extends through a carrier lumen of the electrode carrier (902).

14. A biostimulator system (200), comprising:
a biostimulator transport system (202); and
the biostimulator (100) of any one of claims 1-13.

15. The biostimulator system of claim 14, wherein the biostimulator transport system (202) includes a drive shaft (207) to engage a torque feature (314) of the drive head (310) of the biostimulator (100) of any one of claims 2 to 4.
